Europäisches Patentamt

(19) European Patent Office

Office européen des brevets

(11) Numéro de publication: **0 023 176**
**B1**

(12) **FASCICULE DE BREVET EUROPEEN**

(45) Date de publication du fascicule du brevet: **12.12.84**

(21) Numéro de dépôt: **80401065.0**

(22) Date de dépôt: **16.07.80**

(51) Int. Cl.³: **C 12 M 1/00**, C 02 F 3/28

(54) Installation de fermentation pour la production de méthane à partir de végétaux.

(30) Priorité: **19.07.79 FR 7918991**
**22.08.79 FR 7921421**
**22.11.79 FR 7929204**

(43) Date de publication de la demande:
**28.01.81 Bulletin 81/04**

(45) Mention de la délivrance du brevet:
**12.12.84 Bulletin 84/50**

(84) Etats contractants désignés:
**AT BE CH DE GB IT LI LU NL SE**

(56) Documents cités:
**DE-B-1 010 020**
**DE-C- 884 176**
**DE-C- 905 598**
**FR-A-1 068 501**
**FR-A-1 145 002**

(73) Titulaire: **S.E.T.A.L. Société D'Etudes**
**Techniques Aquitaine-Languedoc**
**130, avenue des Lilas**
**F-64000 Pau (FR)**

(72) Inventeur: **Dehaye, Jean**
**8, rue du Compte de St. Cricq**
**F-64000 Pau (FR)**
Inventeur: **Isman, Marcel**
**6, avenue Joffre**
**F-94100 St. Maur des Fosses (FR)**
Inventeur: **Messud, Pierre**
**Rue des Frères Cousté**
**F-64320 Bizanos (FR)**
Inventeur: **Milois, Jean**
**Résidence Aspin 2**
**F-64000 Pau (FR)**

(74) Mandataire: **Bouju, André**
**38 Avenue de la Grande Armée**
**F-75017 Paris (FR)**

Courier Press, Leamington Spa, England.

### Description

La présente invention concerne une installation de fermentation pour la production de méthane à partir de déchets végétaux divers, tels que fumier, paille et analogue.

Cette installation est particulièrement adaptée pour la mise en oeuvre du procédé de fermentation appelé procédé Ducellier-Isman qui consiste à faire précéder la phase de fermentation anaérobie, d'une phase de préfermentation aérobie dans laquelle on insuffle de l'air au sein de la matière végétale fermentescible.

Les installations connues de ce type fonctionnent en discontinu, c'est-à-dire qu'on les charge successivement en matière fermentescible puis on les décharge en fin de fermentation.

Les installations connues comprennent une cuve de fermentation comportant une ouverture pour introduire à l'intérieur de cette cuve, la matière végétale fermentescible. Ces installations comportent en outre des moyens pour introduire de l'eau à l'intérieur de cette cuve et pour évacuer de cette dernière le liquide chargé de matière solide obtenu en fin de fermentation. En outre, des moyens sont prévus pour aérer l'intérieur de la cuve durant la phase de fermentation aérobie.

Ces cuves sont réalisées en divers matériaux, tels que béton armé, métal, matière plastique rigide telle que polychlorure de vinyle ou poly-ester armé de fibres de verre.

Le chargement de ces cuves en matières végétales fermentescibles est réalisé dans certains cas par pompage d'effluent liquide (par exemple fumier en suspension dans du purin dilué d'eau). Dans un tel procédé de chargement, on limite considérablement la charge en matière végétale solide.

Dans une autre méthode de chargement, on introduit la matière végétale fermentescible par une ouverture de la cuve munie d'un couvercle ou d'une porte, au moyen d'engins de manutention tels que fourche de tracteur agricole, élévateur à bande ou a godet, vis sans fin et analogue, placés à l'extérieur de la cuve.

Ces modes de chargement sont peu pratiques et ne conviennent qu'à des installations de faible capacité de fermentation.

Dans le brevet français 1.068.501, on a proposé une cuve de gazogène à axe horizontal, munie à son extrémité, d'une porte dégageant complètement l'une des quatre faces latérales. Une telle ouverture est bien suffisante pour permettre, par exemple, la pénétration d'une brouette. Mais, selon le texte de ce brevet, cela ne permet pas le chargement normal de la cuve qui doit être terminé à travers un trou d'homme après fermeture de la porte.

Par ailleurs, les cuves connues sont toutes fabriquées en usine, compte tenu des exigences techniques imposées par la nature même de la fermentation (résistance à la corrosion des matériaux, étanchéité aux liquides et aux gaz), et de la nécessité de ce que ces cuves puissent faire l'objet de contrôles très stricts.

On est alors limité en dimension pour ces cuves par les gabarits imposés aux transports routiers ou ferroviaires.

Or, l'intérêt économique de la fermentation méthanique est d'autant plus grand que le volume des cuves de fermentation est plus important. On se trouve donc confronté à un choix entre les deux solutions suivantes:

— construction en usine de cuves de fermentation de qualité satisfaisante, mais de dimensions obligatoirement limitées, donc d'un prix de revient élevé au m³,
— construction sur place de fermenteurs de grande capacité, mais d'une qualité technique discutable sur le plan de l'étanchéité et de la résistance à la corrosion.

Le but de la présente invention est de remédier aux inconvénients des réalisations connues, en créant une installations de fermentation de grande capacité, pouvant ainsi satisfaire aux besoins d'énergie, sous forme de méthane, par exemple d'une grande exploitation agricole, cette installation fonctionnant d'une manière automatique et étant particulièrement pratique à utiliser, en particulier, lors des opérations de chargement et de déchargement.

L'installation de fermentation visée par l'invention comprend une cuve de fermentation de section pratiquement constante sur toute la longueur comportant une porte qui dégage complètement une ouverture correspondant sensiblement à la section de la cuve pour introduire à l'intérieur de cette cuve la matière végétale fermentescible, des moyens pour introduire de l'eau à l'intérieur de cette cuve et pour évacuer de cette dernière le résidu qui est obtenu en fin de fermentation, et des moyens pour aérer l'intérieur de cette cuve, durant la phase de préfermentation aérobie.

Suivant l'invention, cette installation est caractérisée en ce que la cuve est sensiblement parallélépipédique et est constituée d'éléments préfabriqués et comporte une poche étanche ou un revêtement intérieur en matière plastique appliqué sur les parois de cette cuve par projection.

Un premier avantage d'une telle cuve est la suppression des contraintes imposées, dans la fabrication de la cuve, par la nature de la fermentation. En effet, puisque le problème de l'étanchéité est réglé par le revêtement intérieur en matière plastique, indépendamment des problèmes de résistance mécanique, on peut réaliser une cuve suivant l'invention à partir d'éléments préfabriqués qui ne posent pas de problèmes pour leur transport sur route et arriver ainsi à des cuves économiques de très grandes capacités.

Un second avantage découle du premier.

C'est la possibilité d'avoir, compte tenu des dimensions possibles de la cuve une porte qui dégage complètement une ouverture dont les dimensions sont suffisantes pour permettre la pénétration à l'intérieur de la cuve d'un véhicule chargé de matières végétales fermentescibles.

Une telle porte facilite considérablement le chargement et le déchargement de la cuve, puisque le véhicule chargé de matières végétales peut pénétrer entièrement à l'intérieur de la cuve, simplement en roulant sur le sol sur lequel repose la cuve.

Etant donné que les opérations de chargement et de déchargement peuvent être effectuées très rapidement, la cuve peut présenter une très grande capacité en étant ainsi capable de satisfaire complètement aux besoins énergétiques en méthane, pour le chauffage et les machines agricoles à moteur thermique, d'une grande exploitation agricole, ou d'un groupement de petites exploitations.

Dans le cas d'une telle installation, il est préférable que l'ouverture de chargement et de déchargement de la cuve présente une dimension suffisante pour la pénétration à l'intérieur de la cuve d'un tracteur agricole équipé de moyens pour charger et décharger l'intérieur de la cuve.

Toutefois, dans la réalisation préférée de l'invention, on prévoit que ledit véhicule soit constitué par un panier mobile destiné à être chargé à l'extérieur de la cuve par ladite matière végétale fermentescible et à être déplacée à l'intérieur de la cuve par son ouverture, en vue de la fermentation.

Un tel panier peut être facilement chargé à l'extérieur de la cuve de fermentation, puis être introduit à l'intérieur même de cette cuve, par exemple en faisant rouler ce panier sur des rails.

Ainsi, un tel panier peut être poussé à l'intérieur de la cuve, sans que les utilisateurs ou un tracteur agricole entre à l'intérieur de la cuve. On supprime de ce fait les problèmes de pollution ou de risque d'incendie ou d'explosion.

D'autres particularités et avantages de l'invention apparaîtront encore dans la description ci-après.

Aux dessins annexés, donnés à titre d'exemples non limitatifs:

— la figure 1 est une vue en perspective avec arrachements d'une installation de fermentation conforme à l'invention, la porte de la cuve de fermentation étant ouverte,
— la figure 2 est une vue en coupe longitudinale suivant le plan II—II de la figure 1, la porte étant fermée,
— la figure 3 est une vue en perspective, avec arrachements, d'une version préférée de l'installation conforme à l'invention, un panier de chargement et de déchargement étant placé à l'entrée de la cuve de fermentation,

— la figure 4 est une vue en coupe suivant le plan IV—IV de la figure 3, le panier étant en position à l'intérieur de la cuve,

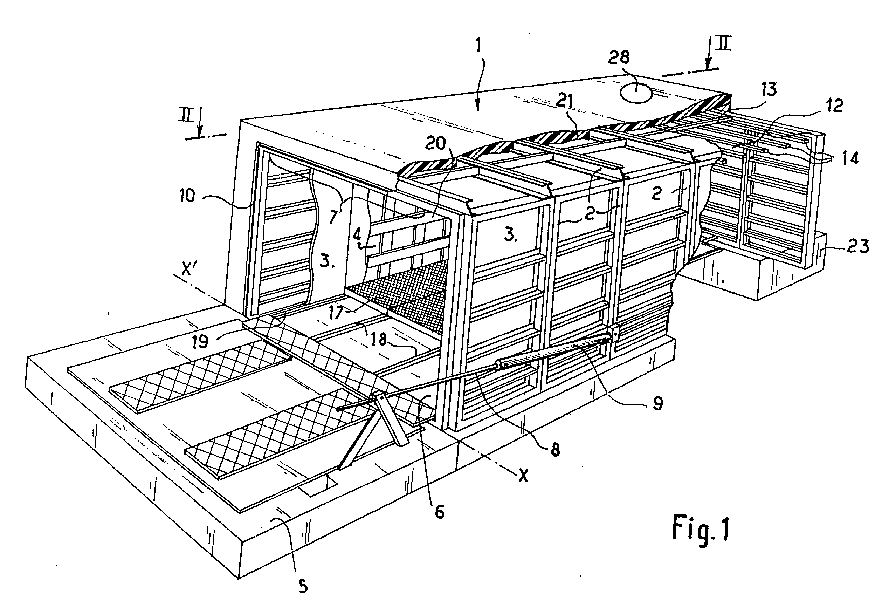

Dans la réalisation de la figure 1, l'installation de fermentation comprend une cuve parallélépipédique 1 comportant une armature constituée par des profilés métalliques 2 assemblés les uns aux autres. Cette armature est revêtue intérieurement par des panneaux en contre-plaqué 3 fixés aux profilés métalliques 2, par exemple par collage. Les profilés métalliques 2 sont protégés contre la corrosion, par exemple par galvanisation à chaud ou par une peinture au zinc.

Cette armature peut être montée sur le lieu même d'une exploitation agricole. Cette armature est tapissée intérieurement par une poche 4, réalisée en une feuille de matière plastique souple, étanche aux gaz de fermentation telle que la caoutchouc naturel ou synthétique ou une matière plastique appropriée. Cette poche 4 est fixée contre les parois latérales, le plancher et le plafond de la cuve 1, par des moyens appropriés tels que des oreilles de fixation non représentées. Cette poche 4 est réalisé selon la technique bien connue des embarcations gonflables.

La cuve 1 comporte une porte 5 qui ouvre complètement l'une de ses faces latérales. Cette porte 5 s'ouvre en basculant vers l'avant, suivant un axe d'articulation X—X', horizontal, situé dans le prolongement du plancher 6 de la cuve 1. En position ouverte, comme indiqué sur la figure 1, la porte 5 constitue un tremplin d'accès à l'intérieur de la cuve 1.

L'ouverture 7 dégagée par la porte 5 a une dimension suffisante pour la pénétration à l'intérieur de la cuve 1 d'un engin de manutention, tel qu'un tracteur agricole équipé d'une machine pour décharger à l'intérieur de la cuve 1 la matière végétale fermentescible.

La structure de la porte 5 est à cet effet, prévue suffisamment solide pour supporter le poids d'un tracteur.

La fermeture et l'ouverture de la porte 5 est commandée par une vis sans fin 8 reliée à la porte 5 et à un système d'écrous 9, à filés trapézoïdaux, reliés à la paroi latérale de la cuve 1, et manoeuvrables de l'extérieur par un vilebrequin.

En position de fermeture, la porte 5 s'applique de façon étanche contre un joint souple 10, fixé autour de l'ouverture 7.

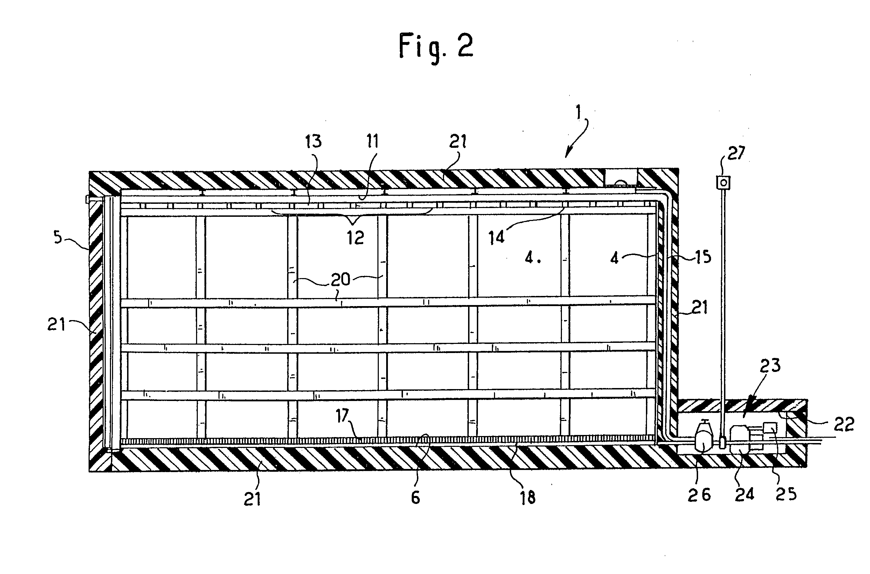

Près du plafond 11 de la cuve 1 (voir figure 2) est située une herse 12 destinée à limiter la hauteur de la matière végétale fermentescible qui est introduite dans la cuve 1. Cette herse 12 est composée d'un tube central 13 relié à une conduite d'admission d'eau 15, et à des tubes transversaux 14 de section carrée munis de trous pour l'arrosage de la matière végétale fermentescible.

Au-dessus du plancher 16 de la cuve 1 est disposé un caillebotis 17. Ce dernier repose sur deux conduites longitudinales 18 et sur des cales latérales en bois 19 (voir figure 1). Ces deux conduites 18 sont prévues pour assurer l'insufflation d'air à l'intérieur de la cuve durant la phase aérobie de la fermentation et pour évacuer le résidu liquide de fermentation.

On voit d'autre part sur les figures 1 et 2 que la poche souple et étanche 4 est protégée contre les chocs éventuels en cours de chargement et de déchargement, par des planches en bois 20 horizontales et verticales, s'étendant sur les parois latérales de la cuve 1, entre le caillebotis 17 et la herse 12.

Par ailleurs, toutes les parois de la cuve 1, y compris la porte 5 et le plancher 16 sont isolées thermiquement par une épaisse couche 21 de matière isolante, telle qu'une mousse de polyuréthane. Cette isolation thermique peut être renforcée en recouvrant de terre les parois latérales fermées de la cuve 1.

A l'extérieur de la cuve 1, à l'opposé de la porte 5, et dans le prolongement du plancher 16, est située une enceinte 22 également isolée thermiquement, renfermant une machinerie 23 comportant tous les organes nécessaires au fonctionnement de la cuve de fermentation 1.

Ces organes comprennent une pompe de circulation 24, un dispositif 25 de réchauffage et de régulation de la température intérieure de la cuve pour maintenir dans cette dernière une température sensiblement égale à 37°C, des vannes d'isolement 26 des différentes conduites et un ventilateur de soufflage 27.

D'autre part, un trou d'homme 28 placé en haut de la cuve 1, permet une mise à l'air libre, ou une circulation d'air forcé à l'intérieur de la cuve 1, avant que l'on pénètre dans cette dernière, en fin de cycle de fermentation.

Grâce à sa structure réalisée à partir d'éléments préfabriqués, la cuve 1 peut être assemblée sur place, sur le lieu même de son exploitation. Cette cuve 1 peut être de très grandes dimensions, en permettant ainsi la production d'une très grande quantité de méthane. On peut de ce fait rendre une grosse exploitation agricole totalement indépendante en matière d'énergie de chauffage ou de fonctionnement des machines agricoles.

Le chargement et le déchargement de la cuve 1 peuvent être réalisés très facilement et très rapidement grâce à la porte 5 qui dégage entièrement l'une de ces faces latérales, en permettant ainsi la pénétration à l'intérieur de la cuve 1, d'un tracteur agricole ou tout autre engin de chargement et de déchargement de grandes dimensions.

Par ailleurs, la poche souple 4 résoud dans des conditions avantageuses au point de vue coût et facilité de mise en oeuvre, les délicats problèmes d'étanchéité de la cuve 1.

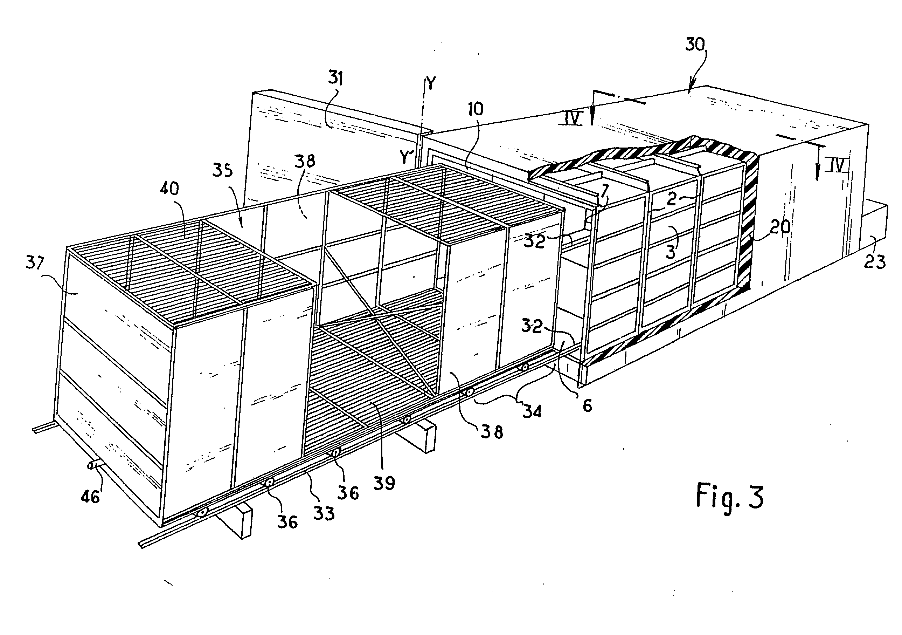

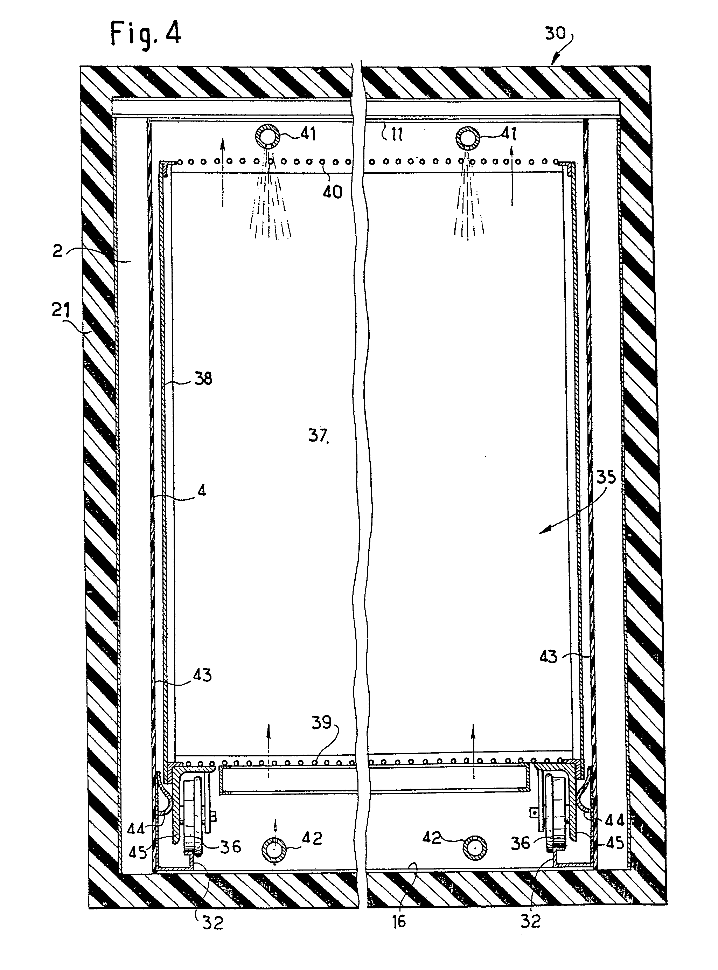

Dans la réalisation des figures 3 et 4, la cuve de fermentation 30 est identique à la cuve 1 représentée sur les figures 1 et 2, sauf en ce qui concerne la porte 31. Dans cette réalisation, les mêmes références numériques sont reprises pour désigner les éléments communs aux cuves 1 et 30.

La porte 31 s'ouvre vers l'avant de la cuve 30 en pivotant suivant un axe Y'—Y vertical, selon un angle supérieur à 90°.

Au-dessus du plancher 16 de la cuve 30, s'étendent deux rails parallèles 32 prolongés à l'extérieur par deux autres rails 33. Les rails extérieurs 33 sont raccordés aux rails intérieur 32 par une portion de rails amovibles 34. Cette dernière peut être enlevée pour permettre l'ouverture et la fermeture de la porte 31.

Sur ces rails 32, 33, 34 est monté un panier 35 mobile sur des galets 36. Ce panier 35 a dimensions qui correspondent sensiblement aux dimensions intérieures de la cuve 30.

Le panier 35 comporte des parois latérales 37, 38 complètement fermées, réalisées à partir de profilés métalliques et de tôles.

La paroi supérieure 39 de même que la paroi inférieure 40 de ce panier 35 sont réalisées en métal déployé comportant une multitude d'ouvertures pour permettre le passage de l'air au travers de ces parois.

La paroi supérieure 39 et les parois latérales 37, 38, sont constituées d'éléments amovibles pour faciliter le chargement et le déchargement du panier 35, par exemple au moyen d'une fourche mécanique portée par un tracteur agricole.

Lorsque le panier 35 est en position à l'intérieur de la cuve 30, comme indiqué sur la figure 4, la paroi supérieure 39 en métal déployé est disposées à une faible distance, sous les tubes d'arrosage 41. La herse de la réalisation, selon les figures 1 et 2 est supprimée, car la fonction de cette dernière est remplie par la paroi supérieure 39 du panier 35 qui limite la hauteur de chargement en matière fermentescible.

Par ailleurs, la paroi inférieure ajourée 40 du panier 35 s'étend au-dessus des conduites 42 d'insufflation d'air, situées au plancher 16 de la cuve 30.

On voit d'autre part, sur la figure 4, que les parois latérales 43 de la cuve 30 comportent chacune un joint souple 44 qui s'applique contre un profilé en équerre 45 qui s'étend le long du panier 35 à l'extérieur et en regard des galets 36. Dans l'exemple représenté, ces joints souples 44 sont réalisé par des bandes de caoutchouc fixées de façon à former un bourrelet à la partie inférieure des parois latérales 43.

La mise en oeuvre de l'installation de fermentation, selon les figures 3 et 4, est réalisée comme suit.

On charge le panier 35 à l'extérieur de la cuve 30 par de la matière végétale fermentescible. Cette opération peut être effectuée très rapidement à l'aide de deux tracteurs équipés de fourches, travaillant de chaque côté du panier 35. Une fois le chargement terminé, le panier 35 est poussé dans la cuve 30 au moyen d'un tracteur. A cet effet, un crochet d'attelage 46 est prévu à l'arrière du panier 35.

On enlève ensuite la portion de rails amovibles 34 et on ferme la porte 31.

Lors de la phase de préfermentation aérobie, on insuffle de l'air par les conduits 42. Cet air traverse de part en part la matière fermentescible en passant par les parois ajourées 39 et 40. Aucune fuite notable d'air n'a lieu latéralement, car les parois latérales 37 et 38 du panier 35 sont complètement fermées et également grâce au joint d'étanchéite 44 prévu à la prtie inférieure des parois latérales 43 de la cuve 30. L'aération de la matière fermentescible est ainsi réalisée dans d'excellentes conditions de rapidité et de rendement. En fin de cycle de fermentation méthanogène, la porte 31 est ouverte, les rails amovibles 34 remis en place et le panier 35 est sorti de la cuve 30 par attelage au crochet 46.

Le résidu de fermentation solide obtenu qui repose sur le fond du panier 35 est déchargé en déposant successivement les éléments amovibles des parois supérieure 40 et latérales 37, 38 du panier 35. Ce résidu est récupéré comme fertilisant.

Le panier 35 facilite par conséquent considérablement le chargement et le déchargement de la cuve 30. Grâce à ce panier, l'intérieur de la cuve 30 reste sensiblement propre après chaque opération. L'entretien de la cuve 30 est de ce fait très réduit. De plus, on évite grâce au panier 35, la pénétration dans la cuve 30 d'un engin à moteur, tel qu'un tracteur qui risque de provoquer des explosions en présence de quantités résiduelles de méthane. On évite également, grâce au panier, la pénétration de personnes à l'intérieur de la cuve 30 où elles sont exposées à des dangers d'explosion et de pollution.

Bien entendu, l'invention n'est pas limitée aux exemples que l'on vient de décrire.

Ainsi, les formes des cuves 1, 30, ainsi que celles du panier 35, peuvent être modifiées, tout en restant parallélépipédiques, pourvu que celles-ci soient sensiblement complémentaires.

Par ailleurs, les cuves 1 et 30, au lieu d'être réalisées en métal peuvent être construites entièrement en bois ou en éléments de béton préfabriqués.

Le panier 35 peut encore comporter un organe moteur permettant son déplacement autonome.

Par ailleurs, chaque installation de fermentation, du type de celle de la figure 3, peut comprendre deux paniers, l'un restant en attente à l'extérieur pendant que l'autre est à l'intérieur de la cuve lors de la fermentation.

La poche 4 peut être remplacée par un revêtement en matière plastique appliquée par projection à l'intérieur de la cuve.

## Revendications

1. Installation de fermentation pour la production de méthane à partir de végétaux divers, comprenant une cuve de fermentation (1), (30), de section pratiquement constante sur toute sa longueur, comportant une porte (5), (31), qui dégage complètement une ouverture correspondant sensiblement à la section de la cuve, pour introduire à l'intérieur de celle-ci la matière végétale fermentescible, des moyens pour introduire de l'eau à l'intérieur de la cuve, des moyens pour en évacuer le résidu obtenu en fin de fermentation et des moyens pour en aérer l'intérieur, caractérisée en ce que ladite cuve (1), (30) est sensiblement parallèlépipédique et est constituée d'éléments préfabriqués (2, 3) et comporte en son intérieur une poche (4) réalisée à partir d'une feuille de matière souple et étanche à l'air et aux gaz formés lors de la fermentation.

2. Installation de fermentation pour la production de méthane à partir de végétaux divers, comprenant une cuve de fermentation (1), (30), de section pratiquement constante sur toute sa longueur, comportant une porte (5), (31), qui dégage complètement une ouverture correspondant sensiblement à la section de la cuve, pour introduire à l'intérieur de celle-ci la matière végétale fermentescible, des moyens pour introduire de l'eau à l'intérieur de la cuve, des moyens pour en évacuer le résidu obtenu en fin de fermentation et des moyens pour en aérer l'intérieur, caractérisée en ce que ladite cuve (1), (30) est sensiblement parallélépipédique et est constituée d'éléments préfabriqués (2, 3) et comporte un revêtement intérieur en matière plastique appliqué sur les parois de cette cuve par projection.

3. Installation conforme à l'une des revendications 1 ou 2, caractérisée en ce que l'ouverture (7) de la cuve (1) a un diamètre suffisant pour la pénétration à l'intérieur de la cuve (1) d'un tracteur agricole équipé de moyens pour décharger à l'intérieur de la cuve ladite matière végétale fermentescible.

4. Installation conforme à l'une des revendications 1 ou 2, la cuve (30) permettant la pénétration en son intérieur d'un véhicule, caractérisée en ce que ledit véhicule est constitué par un panier mobile (35), destiné à être chargé à l'extérieur de la cuve (30) par ladite matière végétale fermentescible et à être déplacé à l'intérieur de la cuve par l'ouverture (7) de la cuve, en vue de la fermentation.

5. Installation conforme à la revendication 4, caractérisée en ce que ledit panier (35) est monté roulant sur des rails (32, 33, 34) s'étendant à l'extérieur et à l'intérieur de la cuve (30).

6. Installation conforme à la revendication 5, la porte (31) étant montée suivant un axe vertical, caractérisée en ce que les rails comprennent une portion amovible (34) adjacente à l'ouverture de la porte, permettant la fermeture de cette dernière après retrait de cette portion amovible.

7. Installation conforme à l'une quelconque des revendications 4 à 6, caractérisée en ce que le panier (35) comporte des parois latérales (37, 38) fermées et des parois supérieure (40) et inférieure (39) munies d'ouvertures d'aération.

8. Installation conforme à la revendication 7, caractérisée en ce que lesdites parois (37, 38, 39, 40) sont constituées d'éléments amovibles.

9. Installation conforme à l'une quelconque des revendications 7 ou 8, caractérisée en ce que près du plancher (16) de la cuve (30) s'étendent des conduites (42) pour insuffler de l'air et pour évacuer le résidu liquide obtenu après fermentation à l'extérieur de la cuve, la paroi inférieure (39) munie d'ouverture d'aération du panier étant située en position de service, au-dessus desdites conduites (42).

10. Installation conforme à la revendication 9, caractérisée en ce que la cuve (30) comporte sur ses parois latérales (43) des moyens (44) pour réaliser un joint sensiblement étanche entre le panier (35) et lesdites parois latérales, lorsque ce panier est en position à l'intérieur de la cuve.

11. Installation conforme à l'une quelconque des revendications 1 à 3, caractérisée en ce que près du plafond de la cuve (1) est située une herse (12) limitant la hauteur de la matière végétale fermentescible introduite dans la cuve.

12. Installation conforme à la revendication 11, caractérisée en ce que cette herse comporte des tubes (14) munis de trous pour arroser d'eau la matière végétale fermentescible.

13. Installation conforme à l'une quelconque des revendications 1 à 12, caractérisée en ce que la cuve (1 30) est calorifugée extérieurement.

## Patentansprüche

1. Gärungsanlage zur Erzeugung von Methan aus verschiedenen vegetarischen Stoffen, mit einem Gärungsbecken (1), (30), das über seine gesamte Länge hinweg einen praktisch konstanten Querschnitt aufweist und mit einer Tür (5), (31) versehen ist, die eine Öffnung vollständig freigibt, die im wesentlichen dem Querschnitt des Beckens entspricht, um die vergärbaren vegetarischen Stoffe in das Becken einzuführen, Mitteln zur Einleitung von Wasser in das Innere des Beckens, Mitteln zum Entnehmen der am Ende der Gärung erhaltenen Reste sowie Mitteln zur Belüftung des Beckeninnenraums, dadurch gekennzeichnet, daß das genannte Becken (1), (30) im wesentlichen quaderförmig und aus vorgefertigten Elementen (2, 3) gebildet ist, sowie innenseitig eine Tasche (4) umfaßt, welche aus einer Folie aus flexiblem Material gebildet ist, welches gegen Luft und die bei der Gärung gebildeten Gase dicht ist.

2. Gärungsanlage zur Erzeugung von Methan aus verschiedenen vegetarischen Stoffen, mit einem Gärungsbecken (1), (30), das über seine gesamte Länge hinweg einen im wesentlichen konstanten Querschnitt aufweist und eine Tür (5), (31) besitzt, welche eine Öffnung vollständig freigibt, die im wesentlichen dem Querschnitt des Beckens entspricht, um in dessen Inneres die vergärbaren vegetarischen Stoffe einzubringen, Mitteln zum Einleiten von Wasser in das Innere des Beckens, Mitteln zum Entnehmen der am Ende der Gärung erhaltenen Reste sowie Mitteln zur Belüftung das Innenraums des Beckens, dadurch gekennzeichnet, daß das genannte Becken (1), (30) im wesentlichen quaderförmig und aus vorgefertigten Elementen (2, 3) gebildet ist sowie eine Innenauskleidung aus einem Plastikmaterial umfaßt, die gegen die Wände dieses Beckens durch Aufschleudern angelegt ist.

3. Anlage nach Anspruch 1 oder 2, dadurch gekennzeichnet, daß die Öffnung (7) des Beckens (1) einen Durchmesser aufweist, der ausreichend groß ist, damit in das Innere des Beckens (1) ein landwirtschaftlicher Traktor eindringen kann, der mit Einrichtungen zum Abladen der genannten vergärbaren vegetarischen Stoffe im Inneren des Beckens ausgestattet ist.

4. Anlage nach Anspruch 1 oder 2, wobei das Becken (30) das Eindringen eines Fahrzeugs in sein Inneres gestattet, dadurch gekennzeichnet, daß das genannte Fahrzeug durch einen beweglichen Korb (35) gebildet ist, welcher dazu bestimmt ist, außerhalb des Beckens (30) mit den vergärbaren vegetarischen Stoffen beladen und in das Innere des Beckens durch dessen Öffnung (7) hindurch bewegt zu werden, damit die Vergärung erfolgt.

5. Anlage nach Anspruch 4, dadurch gekennzeichnet, daß der genannte Korb (35) auf Schienen (32, 33, 34) fahrbar gelagert ist, die sich vom Äußeren in das Innere des Beckens (30) erstrecken.

6. Anlage nach Anspruch 5, bei welcher die Tür (31) um eine senkrechte Achse gelagert ist, dadurch gekennzeichnet, daß die Schienen einen entfernbaren Teil (34) umfassen, der an die Öffnung der Tür angrenzt und das Schließen derselben nach Entfernung dieses entfernbaren Teils gestattet.

7. Anlage nach einem der Ansprüche 4 bis 6, dadurch gekennzeichnet, daß der Korb (35) seitliche geschlossene Wände (37, 38) sowie eine obere (40) und eine untere (39) Wand umfaßt, die mit Belüftungsöffnungen versehen sind.

8. Anlage nach Anspruch 7, dadurch gekennzeichnet, daß die genannten Wände (37, 38, 39, 40) aus entfernbaren Elementen gebildet sind.

9. Anlage nach Anspruch 7 oder 8, dadurch gekennzeichnet, daß in der Nähe des Bodens (16) des Beckens (30) Leitungen (42) verlaufen, um Luft einzublasen und die nach der Gärung erhaltenen flüssigen Reste nach außerhalb des Beckens abzuleiten, wobei die untere, mit Lüftungsöffnungen des Korbes versehene Wand (39) sich in der Betriebsstellung oberhalb der genannten Leitungen (42) befindet.

10. Anlage nach Anspruch 9, dadurch gekennzeichnet, daß das Becken (30) an seinen Seitenwänden (43) Mittel (44) umfaßt, um eine im wesentlichen dichte Verbindung zwischen dem Korb (35) und den genannten Seitenwänden herzustellen, wenn sich der Korb in Stellung im Inneren des Beckens befindet.

11. Anlage nach einem der Ansprüche 1 bis 3, dadurch gekennzeichnet, daß sich in der Nähe der Decke des Beckens (1) eine Egge (12) befindet, welche die Höhe der in das Becken eingeführten vergärbaren vegetarischen Stoffe begrenzt.

12. Anlage nach Anspruch 11, dadurch gekennzeichnet, daß diese Egge Rohre (14) umfaßt, die mit Löchern versehen sind, um die vergärbaren vegetarischen Stoffe mit Wasser zu berieseln.

13. Anlage nach einem der Ansprüche 1 bis 12, dadurch gekennzeichnet, daß das Becken (1, 30) außenseitig wärmegedämmt ist.

## Claims

1. Fermentation installation for production of methane from various plants, comprising a fermentation tank (1), (30) having a practically constant cross-section throughout its length, comprising a door (5), (31) which completely frees an opening corresponding substantially to the cross-section of the tank for introducing the fermentescible vegetable matter within this latter, means for introducing water within the tank, means for removing from this latter the residue obtained at the end of fermentation and means for ventilating the interior of the tank, characterized in that said tank (1), (30) is substantially parallelepipedal and is made up of prefabricated elements (2, 3) and provided internally with a pocket (4) fabricated from a sheet of flexible material which is impervious to air and to the gases formed at the time of fermentation.

2. Fermentation installation for production of methane from various plants, comprising a fermentation tank (1), (30) having a practically constant cross-section throughout its length, comprising a door (5), (31) which completely frees an opening corresponding substantially to the cross-section of the tank for introducing fermentescible vegetable matter within this latter, means for introducing water within the tank, means for removing from this latter the residue obtained at the end of fermentation and means for ventilating the interior of the tank, characterized in that said tank (1), (30) is substantially parallelepipedal and is made up of prefabricated elements (2, 3) and is provided with an internal coating of plastic material applied on the walls of this tank by spraying.

3. Installation in accordance with either of claims 1 or 2, characterized in that the opening (7) of the tank (1) has a sufficiently large diameter to permit entry to the interior of the tank (1) by an agricultural tractor equipped with means for discharging said fermentescible vegetable matter into the interior of the tank.

4. Installation in accordance with either of claims 1 or 2, the tank (30) being such as to permit entry of a vehicle to its interior, characterized in that said vehicle is constituted by a movable basket (35) which is intended to be loaded outside the tank (30) with said fermentescible vegetable matter and to be moved into the interior of the tank through the opening (7) of the tank for the purpose of fermentation.

5. Installation in accordance with claim 4, characterized in that said basket (35) is mounted so as to run on rails (32, 33, 34) which extend outside and inside the tank (30).

6. Installation in accordance with claim 5, the door (31) being mounted along a vertical axis, characterized in that the rails comprise a removable portion (34) which is adjacent to the opening of the door and permits closure of this latter after withdrawal of this removable portion.

7. Installation in accordance with any one of claims 4 to 6, characterized in that the basket (35) has closed side walls (37, 38) and a top wall (40) and bottom wall (39) provided with air vents.

8. Installation in accordance with claim 7, characterized in that said walls (37, 38, 39, 40) are constituted by removable elements.

9. Installation in accordance with either of claims 7 or 8, characterized in that ducts (42) extend close to the floor (16) of the tank (30) for the purpose of blowing air and for the purpose of removing the liquid residue obtained after fermentation to the exterior of the tank, the bottom basket wall (39) provided with an air vent being located above said ducts (42) in the service position.

10. Installation in accordance with claim 9, characterized in that the tank (30) is provided on its side walls (43) with means (44) for forming a substantially leak-tight seal between the basket (35) and said side walls when this basket is in position within the tank.

11. Installation in accordance with any one of claims 1 to 3, characterized in that a cross-bar grating (12) is located near the roof of the tank (1) for limiting the height of fermentescible vegetable matter introduced into the tank.

12. Installation in accordance with claim 11, characterized in that this cross-bar grating comprises tubes (14) provided with holes for sprinkling water onto the fermentescible vegetable matter.

13. Installation in accordance with any one of claims 1 to 12, characterized in that the tank (1, 30) is externally heat-insulated.

Fig.1

0023 176

# Fig. 2

Fig. 3

Fig. 4